Europäisches Patentamt

(19) European Patent Office

Office européen des brevets

(11) Numéro de publication : **0 308 410 B1**

# (12) FASCICULE DE BREVET EUROPEEN

(45) Date de publication du fascicule du brevet :
**16.10.91 Bulletin 91/42**

(21) Numéro de dépôt : **87903351.2**

(22) Date de dépôt : **02.06.87**

(86) Numéro de dépôt international :
**PCT/FR87/00191**

(87) Numéro de publication internationale :
**WO 87/07509 17.12.87 Gazette 87/28**

(51) Int. Cl.⁵ : **A61M 1/00**

(54) **DISPOSITIF D'ACCES ATRAUMATIQUE AU CIRCUIT SANGUIN A FONCTIONNEMENT ROTATIF.**

(30) Priorité : **03.06.86 FR 8607984**

(43) Date de publication de la demande :
**29.03.89 Bulletin 89/13**

(45) Mention de la délivrance du brevet :
**16.10.91 Bulletin 91/42**

(84) Etats contractants désignés :
**AT BE DE FR GB IT LU NL SE**

(56) Documents cités :
**EP-A-01 592 60**
**GB-A- 2 050 175**
**US-A-41 081 73**

(73) Titulaire : **BIOMASYS Société dite:**
**Château de Chailly-sur-Armançon**
**F-21320 POUILLY-EN-AUXOIS (FR)**
Titulaire : **SLONINA, Marie-Noelle**
**10, avenue de la Prise d'Eau**
**F-78110 Le Vésinet (FR)**

Titulaire : **SLONINA, Caroline**
**10, avenue de la Prise d'Eau**
**F-78110 Le Vésinet (FR)**
Titulaire : **SLONINA, Frédéric Arnaud**
**10, avenue de la Prise d'Eau**
**F-78110 Le Vésinet (FR)**
Titulaire : **SLONINA, Aurélie Marianne**
**10, avenue de la Prise d'Eau**
**F-78110 Le Vésinet (FR)**
Titulaire : **HAMMAN, Sylvie**
**66, avenue Aristide Briand**
**F-92000 Levallois (FR)**

(72) Inventeur : **SLONINA, Jean-Pierre**
**(décédé) (FR)**
Inventeur : **HAMMAN, Sylvie**
**66, avenue Aristide Briand**
**F-92000 Levallois (FR)**

(74) Mandataire : **Portal, Gérard et al**
**Cabinet Beau de Loménie 55, rue**
**d'Amsterdam**
**F-75008 Paris (FR)**

## Description

La présente invention concerne un dispositif d'accès atraumatique au circuit sanguin, comportant un dispositif implantable de prélèvement percutané du sang et un mécanisme de commande de l'écoulement sanguin permettant d'effectuer, aussi souvent qu'il est nécessaire une circulation extra-corporelle vers un appareil destiné à compenser un mauvais fonctionnement des reins notamment.

Plusieurs dispositifs d'accès atraumatique au circuit sanguin sont déjà connus et certains utilisent, ainsi que le dispositif objet de l'invention, un corps principal percutané en formé de T, rigide et biocompatible.

Les segments latéraux du T sont chacun reliés à une canalisation artificielle hémocompatible, dont l'une est suturée sur une artère et l'autre sur une veine, de manière à réaliser un shunt artério-veineux constamment parcouru par un courant sanguin.

Le segment central du T, qui traverse la peau, comporte, d'une part une ou plusieurs pièces d'obturation, d'autre part des moyens permettant la fixation d'une tête amovible de prélèvement, reliée au circuit extérieur au corps, et la mise en relation d'au moins une canalisation de prélèvement et une canalisation de réinjection portées par la tête amovible avec le circuit sanguin interne.

Les dispositifs déjà connus ont en commun le fait que la mise en relation des canalisations portées par la tête amovible de prélèvement et de réinjection du sang avec le shunt artério-veineux du circuit sanguin interne se fait soit au moyen d'un déplacement axial d'une pièce d'obturation contenue dans le segment central du T qui traverse la peau soit au moyen de la traversée, dans le sens axial, d'un moyen d'obturation par lesdites canalisations de prélèvement et réinjection.

Au contraire, dans le dispositif objet de la présente invention, la mise en relation des dites canalisations de prélèvement et réinjection portées par la tête amovible avec le shunt artério-veineux a lieu par une rotation simultanée de la tête amovible et de la pièce d'obturation.

L'art antérieur présente un ou plusieurs des principaux inconvénients suivants:

– Nettoyage aléatoire des pièces d'obturation du dispositif percutané en raison de l'accès difficile du désinfectant

– Débits sanguins assez faibles

– Complexité des manoeuvres de branchement et débranchement de la tête amovible, qui nécessitent un outillage compliqué que le malade est incapable d'utiliser correctement avec une seule main.

– Mauvais accrochage externe entre le peau et le corps principal en forme de T, d'où, surtout après des sollicitations par déchirement, un risque de pénétration microbienne.

– Sollicitation mécanique importante d'une pièce en élastomère.

Les principaux avantages du dispositif selon l'invention sont :

– Extrême simplicité d'emploi, pouvant s'effectuer très facilement d'une seule main par le malade.

– Très grande sécurité dans l'asepsie, d'une part car le dispositif implanté ne comporte qu'une seule pièce mobile, en élastomère, très facile à nettoyer, d'autre part en raison de la possibilité d'établir un circuit de désinfection et rinçage pour éliminer le risque de contamination par les microbes contenus dans l'air, enfin grâce à la simplicité de la tête amovible et du bouchon ce qui permet d'en faire des matériels consommables.

– Aucune fatigue mécanique sur la pièce en élastomère.

– Débit sanguin élevé grâce à des sections de passage importantes

– Très bon accrochage entre la peau et le corps principal en forme de T grâce à l'emploi d'une collerette en carbone poreux.

La présente invention possède donc un corps principal tubulaire métallique biocompatible en forme de T dont les segments latéraux sont reliés à un shunt-artério-veineux.

Le segment central du T qui traverse la peau est muni d'une collerette en carbone poreux qui effectue une bonne liaison avec la peau.

Au fond du segment central du T se trouve une pièce d'obturation en élastomère qui est munie d'une collerette destinée à se loger dans une gorge circulaire interne du segment central. Cette gorge positionne axialement la pièce en élastomère et en particulier, maintient sa face inférieure en contact sans interdire la rotation de la pièce.

La pièce d'obturation comprend deux trous ou évidements dont le plan des axes est sensiblement parallèle à son axe de rotation. Ces trous sont en relation avec le shunt artério-veineux lorsqu'il se trouvent placés au-dessus de l'axe de celui-ci et ils en sont isolés de façon suffisamment étanche lorsqu'on effectue une rotation de 90° de la pièce d'obturation. Dans ce cas, ils débouchent en effet sur une face, plane ou de révolution par rapport à l'axe de rotation et solidaire du T, qui coopère parfaitement avec la face inférieure de la pièce d'obturation.

La tête amovible possède une partie cylindrique s'emboîtant parfaitement dans le segment central du T, et elle comprend une canalisation de prélèvement et une canalisation de réinjection.

A l'extrêmité de la tête amovible se trouve une face destinée à appuyer légèrement élastiquement sur le sommet de la pièce d'obturation en élastomère pour obtenir une bonne étanchéité entre les trous de la pièce d'obturation qui coïcident avec les canalisations de la tête amovible.

La tête amovible peut entraîner la pièce d'obturation en rotation par des ergots distincts ou par le prolongement des canalisations de prélèvement et de réinjection du sang qu'elle possède ou part tout autre moyen.

De façon plus précise, l'invention est un dispositif d'accès atraumatique au circuit sanguin du type comportant une pièce métallique tubulaire biocompatible en forme de T, dont les segments latéraux et sont insérés dans un shunt artério-veineux sous-cutané, dont le segment central qui comporte une collerette d'accrochage biologique avec la peau, de préférence en carbone poreux, dépasse légèrement de la surface de la peau, possède une pièce d'oburation et est apte à recevoir et à fixer une tête amovible de prélèvement sanguin et de réinjection comprenant des canalisations de prélèvement sanguin et réinjection communiquant, lors d'un contact entre la tête et la pièce d'obturation, avec des trous ou évidements correspondants de la pièce d'obturation qui se trouvent à ce moment obturés par la face d'étanchéité inférieure du segment central servant d'appui à la face inférieure de la pièce d'obturation, ladite tête comprenant des moyens divers et complémentaires d'entrainement, en rotation commune de la tête et de la pièce d'obturation de sorte que l'exécution d'un mouvement angulaire d'environ 90° par la tête autour de l'axe du segment central fait décrire le même mouvement à la pièce d'obturation, ce qui a pour effet la mise en communication des trous au évidements de la pièce d'obturatian avec le courant sanguin du shunt artério-veineux, soit directement, soit par les trous percés dans la face inférieure du segment central, caractérisé en ce que la tête est amovible relativement à la pièce d'obturation ; et en ce que ladite tête comprend des moyens de fixation de ladite tête de manière amovible sur le sommet du segment central au-dessus du niveau de la peau, ces moyens de fixation étant mis en oeuvre lors de ladite rotation d'environ 90° de la tête.

Les moyens de fixation de la tête amovible sur le sommet du segment central du T, au-dessus du niveau de la peau permettant :

– d'effectuer une rotation de 90° en conservant la même position axiale

– de verrouiller dans une position invariable en fin de course de rotation

– de n'entraîner la pièce d'obturation en rotation que si la position angulaire initiale de la tête amovible avec le segment central du T est correcte.

– d'interdire l'intervention des canalisations de prélèvement et réinjection de la tête amovible par rapport au sens du courant sanguin du shunt artério-veineux.

Les moyens de fixation de la tête amovible sont donc constitués de deux portions circonférentielles de collerette, diamétralement opposées et destinées à s'engager sous deux portions complémentaires et coopérantes de collerette portées par le sommet du segment central du T.

L'épaisseur de la collerette est telle que l'entraînement en rotation de la pièce d'obturation est impossible si les portions de collerette de la tête amovible ne sont pas enfoncées entre leurs homologues du segment central du T. Les portions de collerettes étant dissymétriques, l'intervention des canalisations est impossible. Les portions de collerettes de la tête amovible sont munies de butée et surépaisseur de verrouillage.

La tête amovible possède de surcroît une tige coulissant dans un trou cylindrique parallèle à son axe et dont le rôle est d'obturer un évidement situé à la face inférieure de la tête et reliant les canalisations de prélèvement et de réinjection du sang. Grâce à cet évidement, un nettoyage avec un produit bactéricide et un rinçage sont possibles avant la circulation du sang pour éliminer les bactéries ayant pu subsister dans le segment central du T. Après rinçage, la tige coulissante est abaissée pour isoler les deux futurs courants sanguins et la pièce d'obturation est tournée de 90°.

En raison de la nécessité d'effectuer avec la tête amovible une rotation de 90°, on peut, soit l'équiper de canalisations extérieures suffisamment souples et longues, soit réaliser une pièce non tournante, portant les canalisations extérieures et reliée à deux gorges, circulaires de la partie tournante de la tête. Ces gorges communiquent chacune avec une des canalisations de la tête.

Un dernier point caractéristique de l'invention est le bouchon que l'on met en place dans le segment central du T après usage. Celui-ci épouse en effet toutes les cavités de la pièce d'obturation afin d'interdire la stagnation éventuelle de quelque fluide que ce soit. La position en rotation par rapport au segment central du T est unique en raison de la dissymétrie des portions de collerettes. Le bouchon qui est en élastomère se bloque axialement de manière rédentive sur les portions de collerettes sans mouvement de rotation, ceux-ci étant interdits par sa forme.

L'arrachement du bouchon se fait avec un outillage spécial. De plus, le malade possède une pièce de blocage en rotation du segment central du T pendant la rotation de la tête amovible.

Cette pièce peut être maintenue par l'index du malade pendant que la rotation s'effectue avec le pouce et le majeur. Une seule main suffit donc, ce qui permet notamment au malade d'effectuer lui-même cette opération dans le cas courant où le dispositif percutané est fixé sur son bras gauche.

L'invention sera mieux comprise à l'aide des figures suivantes :

Figure 1 : Vue en coupe du dispositif implantable percutané par l'axe du shunt artério-veineux correspondant à l'axe des segments latéraux du T. Le bouchon est en place.

Figure 2 : Vue en coupe des pièces de la figure 1 observées latéralement, selon une perpendiculaire au shunt artério-veineux ou au plan du T.

Figure 3 : Identique à la figure 2, avec en plus l'arracheur de bouchon et montrant les deux départs du shunt artério-veineux.

Figure 4 : Identique à la figure 1, le bouchon étant retiré.

Figure 5 : Vue extérieure du dispositif implantable par l'axe du segment central du T, le bouchon étant retiré et la collerette en carbone poreux n'étant pas représentée.

Figure 6 : Vue extérieure selon l'axe du segment central du T d'un premier mode de réalisation de la pièce d'obturation.

Figure 7 : Vue latérale en coupe de la pièce d'obturation passant par l'axe des rainures de prélèvement et réinjection.

Figure 8 : Vue latérale extérieure selon un axe perpendiculaire à celui de la figure 7 de la pièce d'obturation.

Figure 9 : Identique à la figure 6 pour un second mode de réalisation de la pièce d'obturation.

Figure 10 : Identique à la figure 7 pour un second mode de réalisation de la pièce d'obturation.

Figure 11 : Identique à la figure 8 pour un second mode de réalisation de la pièce d'obturation.

Figure 12 : Vue en coupe du dispositif implantable par l'axe du shunt artério-veineux, la tête amovible étant en place mais non verrouillée.

Figure 13 : Vue en coupe des pièces de la figure 12 observées latéralement.

Figure 14 : Vue en coupe des pièces de la figure 12, la pièce de blocage en rotation étant en place.

Figure 15 : Vue correspondant à la figure 12, la pièce d'obturation appartenant au second mode de réalisation.

Figure 16 : Vue extérieure selon l'axe du segment central du T lorsqu'elle est en service de la pièce de blocage en rotation.

Figure 17 : Vue extérieure de dessous de la tête amovible selon l'axe des canalisations.

Figure 18 : Vue en coupe de la partie tournante de la tête amovible dans le plan des canalisations.

Figure 19 : Vue en coupe de la partie tournante de la tête amovible dans un plan perpendiculaire au plan des canalisations.

Figure 20 : Vue en coupe dans le plan des canalisations d'un modèle de tête amovible ne possédant que la partie tournante.

Figure 21 : Vue en coupe latérale dans le plan du T du dispositif implantable en ordre de marche, la tête amovible étant verrouillée.

Figure 22 : Vue en coupe des pièces de la figure 21 en ordre de marche, observées selon l'axe du shunt artério-veineux.

Figure 23 : Vue en coupe selon AA.

Figure 24 : Vue en coupe selon l'axe circulaire BB.

Figure 25 : Vue en coupe selon l'axe circulaire CC.

Figure 26 : Vue correspondant à la figure 21, la pièce d'obturation appartenant au second mode de réalisation.

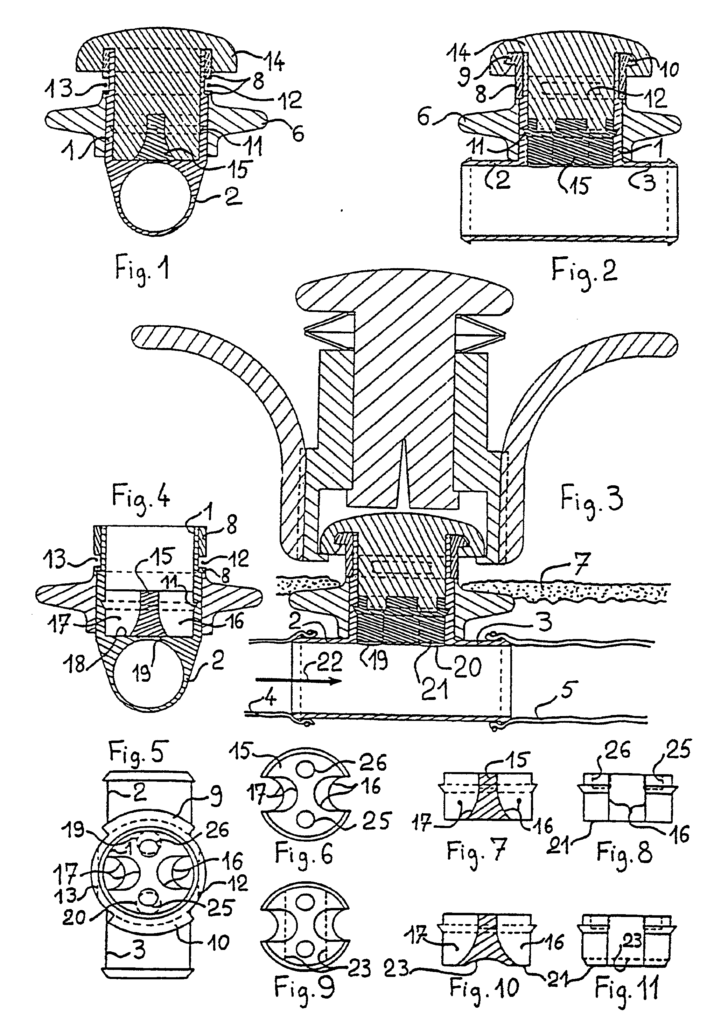

On distingue sur les figures 1 et 2 le segment central 1 du T, métallique biocompatible étant réalisé par exemple en titane ou en acier inoxydable ou en alliage chrome-cobalt. Ce segment central est monobloc avec les segments latéraux 2 et 3, munis de redans permettant l'accrochage visible sur la figure 3 des deux parties 4 et 5 du shunt artério-veineux souple. Ce dernier est réalisé en matière hémocompatible telle que le dacron par exemple.

On voit aussi sur les figures 1 et 2 la collerette 6 en carbone poreux, déjà décrite dans l'art antérieur et permettant un accrochage solide et étanche aux microbes avec la peau 7 visible sur la figure 3.

Le segment central du T possède en son sommet une partie 8 rapportée par collage ou soudage après introduction et collage de la collerette en carbone poreux. Cette partie 8 possède des portions circonférentielles 9 et 10 de collerette visibles sur les figures 2 et 5.

Le segment central possède enfin une gorge circulaire interne 11 permettant le positionnement axial de la pièce d'obturation 15 et deux méplats 12 et 13 visibles sur les figures 1, 2 et 5 notamment, permettant la mise en place de la pièce de blocage en rotation 40.

Sur les figures 1 et 2, le bouchon 14 est en place et on voit qu'il pénètre dans tous les trous de la pièce d'obturation 15. Ces deux dernières pièces 14 et 15 sont de préférence réalisées en matière élastomère biocompatible telle que le polyuréthane par exemple. La collerette circulaire de la pièce 15 pénètre dans la gorge circulaire interne 11 grâce à l'effet élastomère. L'emploi d'un élastomère n'est cependant pas limitatif de l'invention bien qu'il offre de nombreux avantages pour l'étanchéité et qu'il nécessite des pièces moins précises.

En effet, le bouchon 14 pourrait en perdant un peu de son efficacité, ne pas pénétrer dans la pièce 15 et de ce fait pouvoir tourner et se verrouiller, comme la tête amovible sur les portions circonférentielles de collerette 9 et 10.

Par ailleurs, la pièce d'obturation pourrait, en perdant aussi de son efficacité, être maintenue en place par un jonc métallique ou de plastique.

La figure 3 explique le fonctionnement très simple d'un type non limitatif d'arracheur de bouchon n'exerçant aucun effort sur la peau puisque la traction, et la compression qui l'annulent, sont exercées par l'index, la majeur et le pouce respectivement.

Après arrachement du bouchon, le dispositif implantable se trouve dans la position de la figure 4, c'est-à-dire avec sa pièce d'obturation 15 en position fermée. Les cavités ou trous 16 et 17 de celle-ci se trouvent en effet limitées par la face plane ou de révolution 18 constituant le fond du segment central du T. Dans ce cas, les deux trous 19 et 20 d'accès au shunt artério-veineux sont donc obturés par la face inférieure 21 de la pièce d'obturation. Cette face 21 ainsi que la face coopérante 18 solidaire du corps principal en T peut se trouver, selon l'invention, à plusieurs niveaux par rapport au sommet de l'alésage des segments latéraux 2 et 3 du T.

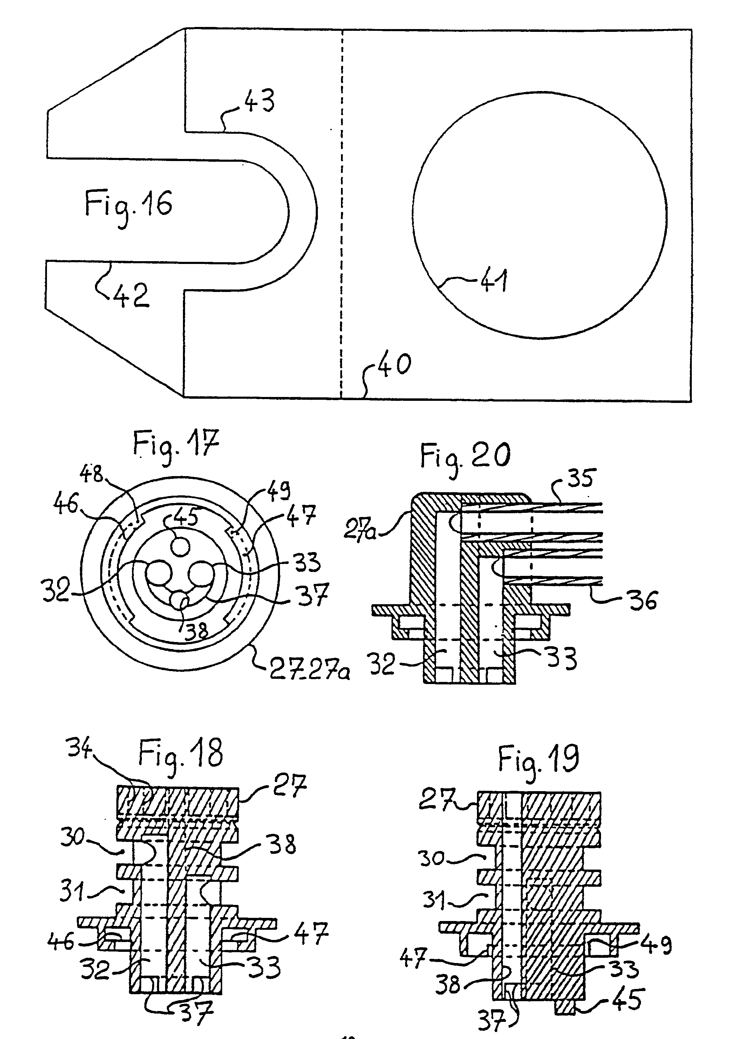

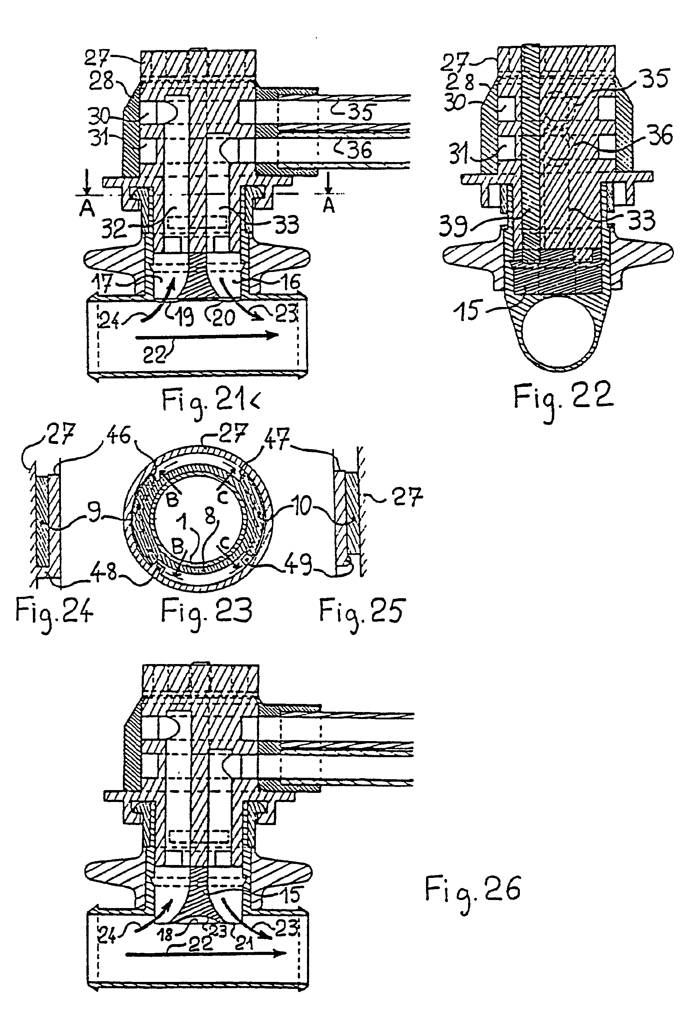

Par exemple, sur les figures 1 à 4, ce niveau se trouve légèrement au-dessus du sommet de l'alésage, de sorte qu'il existe une très mince paroi à franchir au moyen des trous 19 et 20. Mais sur les figures 15 et 26, ce niveau se trouve au contraire au-dessous du sommet de l'alésage des segments latéraux 2 et 3 du T. Les trous 19 et 20 deviennent alors inutiles puisque lapièce 15 et ses trous ou cavités 16 et 17 plongent directement dans le flux sanguin 22, en position de marche.

La face inférieure 21 de la pièce 15 comprend dans ce cas une partie centrale 23 dont la concavité correspond à la courbure des alésages des segments 2 et 3, de sorte qu'en l'absence de prélèvement sanguin que l'on peut voir sur la figure 15, la forme de l'alésage n'est pas altérée.

L'intérêt de l'abaissement du niveau de la face 21 au dessous du sommet des alésages des segments 2 et 3 n'existe que si le courant sanguin 22 est trop faible par rapport au courant de réinjection 23 et que l'on peut craindre qu'une partie de ce courant se mélange au courant de prélèvement 24.

Les pièces d'obturation 15 correspondant à ces deux types de réalisations sont représentées sur les figures 6 à 8 et 9 à 11 respectivement. On voit qu'elles ne diffèrent que par leur face inférieure. Dans les deux types de réalisation, les trous ou évidements pour le prélèvement et la réinjection 16 et 17 ont un profil très incliné pour favoriser la réinjection 23 du sang dans le sens du courant 22.

La pièce d'obturation 15 possède enfin deux trous borgnes 25 et 26 d'entraînement en rotation par la tête amovible. Ceux-ci ne sont pas limitatifs de l'invention car il y a de très nombreuses façons d'entraîner cette pièce. En particulier, l'entraînement pourrait avoir lieu par les trous 16 et 17 ou par une fente pour tournevis, etc.

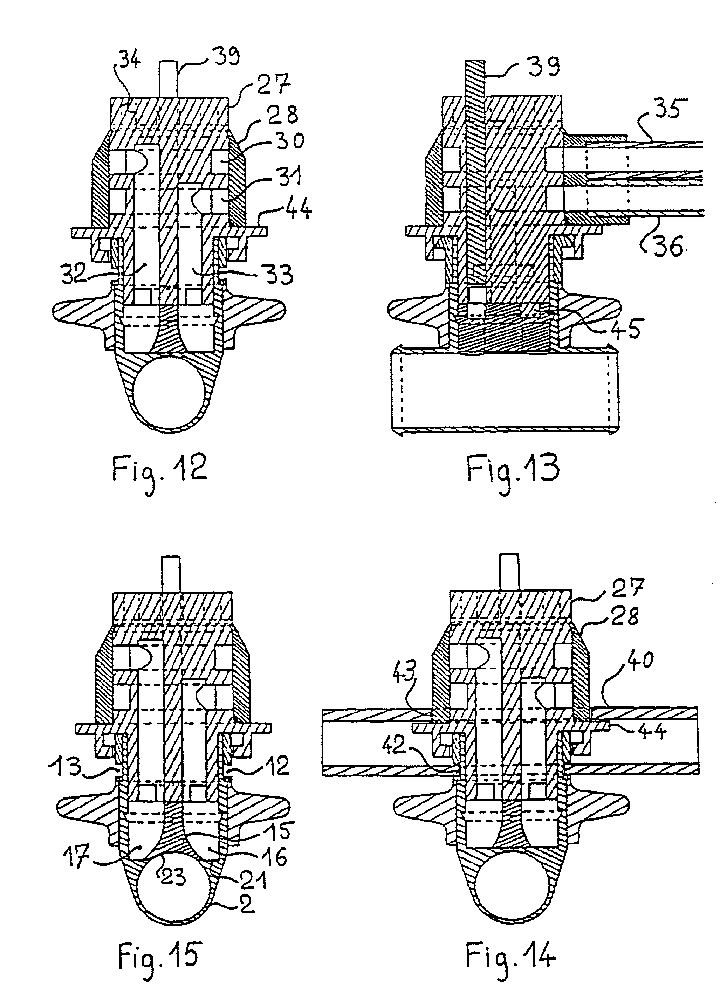

Sur la figure 12, la tête amovible 27 est en place. Celle-ci comprend ici une pièce non tournante 28. La pièce 28 est ajustée de façon suffisamment étanche sur la pièce tournante 27 et immobilisée axialement par le redan 29. Il est donc avantageux, quoique non obligatoire, que la pièce 28 ait une assez bonne élasticité. De nombreuses matières plastiques peuvent convenir, telles que Delrin, Lexan, polyuréthane, etc...

Le pièce tournante 27 comprend deux rainures circulaires 30 et 31 visibles sur les figures 12, 18 et 19 en particulier. Ces rainures communiquent respectivement avec les canalisations 32 et 33 de prélèvement et de réinjection de la pièce 27.

Le sommet de la pièce tournante 27 possède des aspérités 34 qui favorisent l'entraînement en rotation par les doigts.

Sur la figure 20, on voit une autre forme de tête amovible dont la pièce tournante 27a est directement reliée aux canalisations extérieures souples 35 et 36 communicant avec les canalisations 32 et 33 respectivement.

Sur la figure 17, on voit la tête amovible 27 ou 27a par le dessous, c'est-à-dire par la face qui va s'enfoncer dans le segment central du T et venir au contact avec la pièce d'obturation 15 en comprimant légèrement celle-ci axialement.

On voit sur la face inférieure de la pièce 27 un évidement circulaire 37 qui relie les canalisations 32 et 33. Sur le trajet de cet évidement 37 se trouve un trou cylindrique 38, parallèle à l'axe de la tête 27 et qui est parcouru par une tige coulissante 39 visible sur les figures 12 et 13 notamment. On voit sur ces figures que lorsque la tige 39 est relevée, il y a communication entre les canalisations 32 et 33 par l'évidement 37, ce qui permet un lavage et un rinçage sans réexposition à l'air de toutes les zones qui seront en contact avec le sang.

Cette communication est supprimée par abaissement de la tige 39 avant d'effectuer la rotation de 90° de la pièce 27 qui mettra ses canalisations 32 et 33 en contact avec le sang.

La présence de l'évidement 37 et de la tige 39 n'est pas obligatoire donc non limitative de l'invention.

La figure 14 indique le mode de fonctionnement de la pièce 40 de blocage en rotation du segment central du T pendant la rotation de la tête 27.

La pièce 40 est également visible sur la figure 16 et l'on distingue son trou 41 destiné à recevoir l'index du malade qui recevra l'effort de rotation.

On voit aussi une gorge inférieure 42 qui vient s'engager dans les méplats 12 et 13 du segment central 1 du T pour l'empêcher de tourner.

On voit enfin une gorge supérieure 43 dont le rôle est d'entourer la pièce non tournante 28 et d'empêcher le soulèvement de la pièce tournante 27 qui vient en butée par sa corolle circulaire 44 sous la gorge 43.

L'opération de lavage et de rinçage interne s'effectue donc avec la tête amovible 27 en place mais non verrouillée, étant simplement maintenue par la pièce de blocage 40 qui la fixe provisoirement au dispositif implantable.

Quant ces opérations rapides sont achevées, le malade appuie à fond sur la tige 39 qui vient s'encastrer dans le trou borgne 26 de la pièce d'obturation 15 afin d'obturer l'évidement circulaire 37 et de participer à l'entraînement en rotation de la pièce 15. L'autre moyen d'entraînement en rotation de la pièce 15 est l'ergot 45 Porté par la face inférieure de la pièce 27 et qui s'enfonce dans le trou borgne 25 de la pièce d'obturation 15.

La hauteur de l'ergot d'entraînement 45 est légèrement inférieure à la somme des épaisseurs des portions circonférentielles de collerettes 9 et 46 d'une part ou 10 et 47 d'autre part, de telle sorte que l'ergot 45 ne peut entraîner la pièce 15 en rotation que si les portions circonférentielles de collerettes respectivement solidaires de la tête amovible 27 ou 27a et de la pièce 8 solidaire du segment central 1 sont emboîtées axialement.

Puis le malade enfonce son index dans le trou 41 de la pièce de blocage 40 et saisit le sommet de la pièce 27 entre son pouce et son index pour le faire tourner de 90° dans le sens des aiguilles d'une montre. (Sens correspondant aux dessins mais évidemment pas limitatif de l'invention). L'appareil est alors en ordre de marche, comme indiqué sur les figures 21 à 26 et le malade retire la pièce de blocage 40 qui est relativement encombrante.

Sur la pièce 21, on voit donc le flux de prélèvement sanguin 24 qui s'engage dans le trou 19 puis le trou ou évidement 17 puis la canalisation 32, la gorge 30 et la canalisation souple extérieure 35 vers l'appareil de traitement. Le flux de réinjection provient de la canalisation 36, la gorge 31, la canalisation 33, le trou ou évidement 16, le trou 20 et arrive en 23 avec une orientation favorable par rapport à l'écoulement sanguin permanent 22.

Sur la figure 23 et ses rabattements en figure 24 et 25, on voit en coupes AA, BB et CC, en position montée, les positions respectives des portions circonférentielles de collerette 9 et 10 portées par la pièce 8 solidaire du segment central 1 et des portions circonférentielles de collerette 46 et 47 portées par la tête amovible 27 ou 27a.

Les portions circonférentielles de collerette 46 et 47 portées par la pièce 27 possèdent respectivement une butée 48 et une surépaisseur de verrouillage 49 visibles sur les figures 17, 23, 24, 25.

Cette disposition n'est pas limitative de l'invention car chaque portion circonférentielle de collerette pourrait posséder à la fois en butée angulaire et une surépaisseur de verrouillage et par ailleurs, les butées et verrouillages pourraient, à l'inverse, être solidaires des portions circonférentielles de collerettes 9 et 10.

La pièce 27 étant de préférence en plastique biocompatible du type PTFE, polyethylène, Delrin, polycarbonate, etc ... supporte aisément une flexion élastique de la portion circonférentielle de collerette 47 pour permettre le passage de la surépaisseur de verrouillage 49.

Pour le démontage après usage, le malade réintroduit la pièce de blocage 40 dans les méplats 12 et 13 et tourne de 90° en sens inverse la tête amovible 27.

Cette opération rétablit l'obturation des trous 19 et 20 par la pièce 15. Ensuite, le malade retire la pièce 40, enlève la tête 27 et met un bouchon 14 en place.

Il est très souhaitable pour la commodité de l'opération par le malade lui-même que le bouchon 14 et la tête amovible 27a, 27 avec la partie non tournante éventuelle 28 et les canalisations extérieures souples 35 et 36 soient des pièces jetables après usage car cela garantit une meilleure stérilité. Le dessin des pièces selon l'invention permet d'obtenir ces éléments à très bon marché, donc on peut les considérer à juste titre comme des pièces consommables.

L'invention n'est pas limitée aux seules formes ou moyens décrits et représentés. Des formes équivalentes ou des moyens différents mais évidents pour l'homme de l'art et dont la mise en oeuvre n'apporterait pas une réelle activité inventive font partie de l'invention dont la portée est précisée par les revendications suivantes :

## Revendications

1. Dispositif d'accès atraumatique au circuit sanguin du type comportant une pièce métallique tubulaire biocompatible en forme de T, dont les segments latéraux (2) et (3) sont insérés dans un shunt artério-veineux (4) (5) sous-cutané, dont le segment central (1) qui comporte une collerette (6) d'accrochage biologique avec la peau, de préférence en carbone poreux, dépasse légèrement de la surface de la peau (7), possède une pièce d'obturation (15) et est apte à recevoir et à fixer une tête amovible (27) (28), (27a) de prélèvement sanguin et de réinjection comprenant des canalisations de prélèvement sanguin (32) et réinjection (33) communiquant, lors d'un contact entre la tête et la pièce d'obturation (15), avec des trous ou évidements (17) (16) correspondants de la pièce d'obturation (15) qui se trouvent à ce moment obturés par la face d'étanchéité inférieure (18) du segment central (1) servant d'appui à la face inférieure (21) de la pièce d'obturation (15), ladite tête comprenant des moyens divers et complémentaires d'entraînement (39) (45), (25) (26), en rotation commune de la tête et de la pièce d'obturation (15) de sorte que l'exécution d'un mouvement angulaire d'environ 90° par la tête (27) (28), (27a) autour de l'axe du segment central (1) fait décrire le même mouvement à la pièce d'obturation (15), ce qui a pour effet la mise en communication des trous ou évidements (16) (17) de la pièce d'obturation (15) avec le courant sanguin (22) du shunt artério-veineux, soit directement, soit par les trous (19) (20) percés dans la face inférieure (18) du segment central (1), caractérisé en ce que la tête (27), (28), (27a) est amovible relativement à la pièce d'obturation (15) ; et en ce que ladite tête (27), (28), (27a) comprend des moyens de fixation (46), (47) de ladite tête de manière amovible sur le sommet du segment central (1) au-dessus du niveau de la peau, ces moyens de fixation étant mis en oeuvre lors de ladite rotation d'environ 90° de la tête (27), (28), (27a).

2. Dispositif selon la revendication 1, caractérisé en ce que la pièce d'obturation (15) est en élastomère hémocompatible, par exemple le polyuréthane.

3. Dispositif selon les revendications 1 et 2, caractérisé en ce que la pièce d'obturation (15) est positionnée axialement dans le segment central (1) du T grâce à une petite collerette qui vient se loger élastiquement dans une gorge circulaire interne (11) dudit segment central.

4. Dispositif selon l'une des revendications 1 à 3, caractérisé en ce que le profil des trous ou évidements (16) et (17) de la pièce d'étanchéité (15) est nettement incliné vers le bas et l'extérieur de manière à favoriser la séparation du flux sanguin de réinjection (23) du flux sanguin de prélèvement (24).

5. Dispositif selon la revendication 1, caractérisé en ce que la face, d'extrémité inférieure de la tête amovible (27) (28), (27a) qui vient au contact du sommet de la pièce d'étanchéité (15), possède un évidement (37) qui relie entre elles les canalisations de prélèvement (32) et de réinjection (33) de ladite tête amovible, ledit évidement (37) pouvant être obturé par l'abaissement d'une tige (39) coulissant dans un logement (38) sensiblement parallèle à l'axe de la tête amovible de telle sorte qu'il est possible, lorsque la tête amovible est en place, mais avant de la faire tourner de 90° pour mettre le dispositif en position de fonctionnement, d'effectuer un lavage et un rinçage à l'abri de l'air de l'ensemble des volumes qui seront concernés par le sang.

6. Dispositif selon la revendication 1, caractérisé en ce que les moyens de fixation de la tête amovible (27) (28), (27a) comprennent deux portions circonférentielles de collerette (46) (47) qui pénètrent axialement entre deux portions circonférentielles de collerette complémentaires (9) (10) solidaires du sommet du segment central (1) du T et viennent se glisser sous lesdites portions complémentaires par le mouvement de rotation précité.

7. Dispositif selon la revendication 6 caractérisé en ce que la pénétration axiale des portions circonférentielles de collerette solidaires de la tête amovible (27) (28), (27a) dans leurs homologues complémentaires (9) (10) solidaires du sommet du segment central (1) du T n'est possible que pour une seule position angulaire, grâce à une dissymétrie, de manière à empêcher l'interversion accidentelle des canalisations de prélèvement et de réinjection et que par ailleurs l'action des moyens d'entraînement en rotation (39) (45) de la pièce d'obturation (15) par la tête (27) (28), (27a) n'est possible que s'il y a eu pénétration axiale entre lesdites portions circonférentielles de collerette.

8. Dispositif selon la revendication 6 caractérisé en ce que les portions circonférentielles de collerettes (46) (47) solidaires de la tête (27) (28), (27a) ou solidaires du segment central (1) du T comportent respectivement au moins une butée angulaire (48) limitant la rotation à la valeur exacte choisie et une surépaisseur de verrouillage (49).

9. Dispositif selon la revendication 1 caractérisé en ce que pendant la rotation simultanée de la tête amovible (27) (28), (27a) et de la pièce d'obturation (15) qui tend à entraîner dans le même sens l'ensemble de la partie implantable du dispositif, une pièce de blocage en rotation (40) du segment central (1) du T munie d'une gorge (42) est introduite autour de deux méplats (12) et (13) de la pièce (8) solidaire de celui-ci, ladite pièce de blocage étant immobilisée par un trou (41) ouvert ou fermé dans lequel le malade peut introduire un doigt, l'index de préférence. En position d'enfoncement maximal de la pièce de blocage (40), une deuxième gorge (43) de celle-ci vient ceinturer le sommet de la corolle circulaire (44) solidaire de la tête amovible (27) (28), (27a) de manière à interdire le recul axial de ladite tête pendant les opérations s'effectuant avant son verrouillage.

10. Dispositif selon la revendication 9 caractérisé en ce que la tête amovible est constituée d'une seule pièce (27a) à laquelle sont collées deux canalisations extérieures (35) et (36) suffisamment souples pour accepter la rotation de 90° pendant la phase de verrouillage, ou de deux pièces (27) (28) dont la première (27), destinée à tourner de 90° dans le segment central (1) du T pendant le verrouillage sous l'effet d'un mouvement exercé par les doigts sur les aspérités (34) de son sommet possède deux gorges circulaires (30) et (31) chacune reliée à une de ses deux canalisations de prélèvement (32) et réinjection (33) et dont la deuxième (28) est reliée aux deux canalisations extérieures (35) et (36) et permet la rotation en son centre de la partie tournante (27) tout en ménageant une étanchéité suffisante entre la canalisation (35) et la gorge (30) et entre la canalisation (36) et la gorge (31).

11. Dispositif selon l'une des revendications précédentes caractérisé en ce qu'un bouchon (14) en matière élastomère de préférence biocompatible telle que le polyuréthane par exemple, a une forme telle qu'une fois mis en place, il ne laisse aucun espace vide dans les cavités de la pièce d'obturation (15) de manière à éviter la stagnation de tous fluides.

## Claims

1. Device for atraumatic access to the blood circuit, of the type comprising a tubular, metallic, biocompatible part in the form of a T of which the lateral segments (2) and (3) are inserted in a sub-cutaneous arterio-venous shunt (4), (5), of which the central segment (1) which comprises a flange (6) for biological catching with the skin, preferably made of porous carbon, projects slightly from the surface of the skin (7), possesses an obturation part (15) and is adapted to receive and to fix a removable blood taking and reinjection head (27) (28), (27a), comprising blood taking (32) and reinjection (33) lines which communicate whenever the head contacts with the obturation part (15), with corresponding holes or recesses (17) (16) of the obturation part (15) which are at that moment obturated by the lower sealing face (18) of the central segment (1) serving as support for the lower face (21) of the obturation part (15); said head comprising various and complementary drive means (39) (45), (25) (26), in joint rotation of the head and obturation part (15) so that any angular movement of the head (27) (28), (27a) of about 90°, around the axis of central segment (1) imparts the same movement to the obturation part (15), this having for effect the placing of the holes or recesses (16 (17) of the obturation part (15) in communication with the flow of blood (22) of the arterio-venous shunt, either directly, or via the holes (19) (20) made in the lower face (18) of the central segment (1), characterized in that the head (27), 28), (27a) is removable relatively to the obturation part (15); and in that said head (27), (28), (27a) comprises means (46), (47) of removably fixing said head on the top of the central segment (1) above the level of the skin, said fixing means being applied during said rotation of about 90° of the head (27), (28), (27a).

2. Device according to claim 1, characterized in that the obturation part (15) is made of haemocompatible elastomer, for example polyurethane.

3. Device according to claims 1 and 2, characterized in that the obturation part (15) is positioned axially in the central segment (1) of the T thanks to a small flange which is elastically housed in an inner circular groove (11) in said central segment

4. Device according to one of claims 1 to 3, characterized in that the profile of the holes or recesses (16) and (17) of the sealing part (15) is clearly inclined downwardly and outwardly so as to promote separation of the reinjection blood flow (23) from the taking blood flow (24).

5. Device according to claim 1, characterized in that the lower end face of the removable head (27) (28), (27a) which comes into contact with the top of the sealing part (15) possesses a recess (37) which connects together the taking (32) and reinjection (33) lines of said removable head, said recess (37) being able to be obturated by lowering a rod (39) sliding in a housing (38) substantially parallel to the axis of the removable head so that it is possible, when the removable head is in place, but before rotating it through 90° to place the device in position of operation, to effect an air-free washing and rinsing of all the volumes which will be concerned by the blood.

6. Device according to claim 1, characterized in that the fixing means of the removable head (27) (28), (27a) comprise two circumferential portions of flange (46) (47) which penetrate axially between two complementary circumferential portions of flange (9) (10) fast with the top of the central segment (1) of the T and slide under said complementary portions by said movement of rotation.

7. Device according to claim 6, characterized in that the axial penetration of the circumferential portions of flange fast with the removable head (27) (28), (27a) in their complementary homologues (9) (10) fast with the top of the central segment (1) of the T is possible only for one angular position, thanks to a dissymetry, so as to prevent accidental inversion of the taking and reinjection lines and furthermore the action of the means (39) (45) for driving in rotation the obturation part (15) by the head (27) (28), (27a) is possible only if there has been axial penetration between said circumferential portions of flange.

8. Device according to claim 6, characterized in that the circumferential portions of flanges (46) (47) fast with the head (27) (28), (27a) or fast with the central segment (1) of the T respectively comprise at least one angular stop (48) limiting the rotation to the exact value chosen and an excess thickness (49) for locking.

9. Device according to claim 1, characterized in that, during simultaneous rotation of the removable head (27) (28), (27a) and of the obturation part (15) which tends to drive in the same direction the whole of the implantable part of the device, a part (40) for blocking in rotation the central segment (1) of the T provided with a groove (42) is introduced around two flat portions (12) and (13) of the part (8) fast therewith, said blocking part being immobilized by a hole (41), open or closed, in which the patient can introduce a finger, preferably the index finger. In position of maximum penetration of the blocking part (40), a second groove (43) therein surrounds the top of the circular corolla (44) fast with the removable head (27 (28), (27a) so as to prevent axial recoil of said head during the operations effected before locking thereof.

10. Device according to claim 9, characterized in that the removable head is constituted by a single piece (27a) to which are adhered two outside lines (35) and (36) sufficiently supple to accept rotation through 90° during the locking phase, or by two parts (27) (28) of which the first (27), intended to rotate through 90° in the central segment (1) of the T during locking under the effect of a movement exerted by the fingers on the rough parts (34) of its top, possesses two circular grooves (30) and (31) each connected to one of its two taking (32) and reinjection (33) lines and of which the second (28) is connected to the two outside lines (35) and (36) and allows the rotation at its centre of the rotating part (27), whilst forming a sufficient tightness between line (35) and groove (30) and between line (36) and groove (31).

11. Device according to one of the preceding claims, characterized in that a stopper (14) made of elastomer matter, preferably biocompatible such as polyurethane for example, has such a shape that, once placed in position, it allows no empty space in the cavities of the obturation part (15) so as to avoid stagnation of any fluids.

## Patentansprüche

1. Vorrichtung für den atraumatischen Zutritt zum Blutkreislauf mit einem biokompatiblen T-förmigen Rohrstück aus Metall, dessen seitliche Abschnitte (2) und (3) in einem subkutanen arteriovenösen Shunt (4) (5) eingesetzt sind, dessen mittlerer Abschnitt (1), der einen Bund (6), vorzugsweise aus porösem Kohlenstoff, zum biologischen Anhaften an der Haut aufweist; von der Oberfläche der Haut (7) geringfügig vorsteht, ein Verschluss-Stück (15) besitzt und zur Aufnahme und Befestigung eines abnehmbaren Kopfes (27) (28) (27a) für die Blutentnahme und Reinjektion mit Leitungen für die Blutentnahme (32) und für die Reinjektion (33) ausgelegt ist, welche bei einem Kontakt zwischen Kopf und Verschluss-Stück (15) mit entsprechenden Löchern oder Ausnehmungen (17) (16) des Verschluss-Stücks (15) kommunizieren, welche zu diesem Zeitpunkt durch die untere Dichtseite (18) des als Auflage für die Unterseite (21) des Verschluss-Stücks (15) dienenden mittleren Abschnitts (1) verschlossen sind, wobei der Kopf verschiedene und komplementäre Antriebsmittel (39) (45), (25) (26) für die gemeinsame Rotation des Kopfes und des Verschluss-Stücks (15) umfasst, sodass die Ausführung einer Winkelbewegung um etwa 90° des Kopfes (27) (28), (27a) um die Achse des mittleren Abschnitts (1) dieselbe Bewegung vom Verschluss-Stück (15) beschreiben lässt, wodurch Löcher oder Ausnehmungen (16) (17) des Verschluss-Stücks (15) mit dem Blutstrom (22) des arteriovenösen Shunt entweder direkt oder über die in die Unterseite (18) des mittleren Abschnitts (1) gebohrten Löcher (19) (20) in Kommunikation versetzt werden, dadurch gekennzeichnet, dass der Kopf (27) (28), (27a) gegenüber dem Verschluss-Stück (15) abnehmbar ist und dass der Kopf (27) (28), (27a) Mittel (46), (47) zur abnehmbaren Befestigung des Kopfes auf dem obersten Teil des mittleren Abschnitts (1) oberhalb des Niveaus der Haut umfasst, welche Befestigungsmittel bei einer Rotation des Kopfes (27) (28), (27a) um etwa 90° in Funktion gesetzt werden.

2. Vorrichtung nach Anspruch 1, dadurch gekennzeichnet, dass das Verschluss-Stück (15) aus einem hemokompatiblen Elastomer, beispielsweise Polyurethan, besteht.

3. Vorrichtung nach den Ansprüchen 1 und 2, dadurch gekennzeichnet, dass das Verschluss-Stück (15) mit einem kleinen Bund, der elastisch in einer kreisrunden Innennut (11) des mittleren Abschnitts zu liegen kommt, axial im mittleren Abschnitt (1) des T positioniert ist.

4. Vorrichtung nach einem der Ansprüche 1 bis 3, dadurch gekennzeichnet, dass das Profil der Löcher oder Ausnehmungen (16) und (17) des Dichtstücks (15) deutlich nach unten und nach aussen geneigt ist, sodass die Trennung des Blutstroms für die Reinjektion (23) vom Entnahmeblutstrom (21) begünstigt ist.

5. Vorrichtung nach Anspruch 1, dadurch gekennzeichet, dass die Seite des unteren Endes des abnehmbaren Kopfes (27) (28), (27a), die mit dem obersten Teil des Dichtstücks (15) in Kontakt gelangt, eine Ausnehmung (37) besitzt, die die Entnahme- (32) und Reinjektions- (33) Leitungen des abnehmbaren Kopfes miteinander verbindet und welche Ausnehmung (37) durch Herabsenken einer Stange (39), welche in einer zur Achse des abnehmbaren Kopfes im wesentlichen parallelen Aufnahme (38) gleitend gelagert ist, verschlossen werden kann, sodass es, wenn sich der abnehmbare Kopf in seiner Lage befindet, aber vor Drehen desselben um 90° zwecks Positionierens der Vorrichtung in die Funktionsstellung möglich ist, eine Waschung und Spülung aller vom Blut betroffenen Volumen unter Luftabschluss vorzunehmen.

6. Vorrichtung nach Anspruch 1, dadurch gekennzeichnet, dass die Befestigungsmittel des abnehmbaren Kopfes (27) (28), (27a) zwei Umfangsteile (46) (47) umfassen, welche axial zwischen zwei komplementäre Umfangsbundteile (9), (10), die mit dem obersten Teil des mittleren Abschnitts (1) des T verbunden sind, dringen und durch die Rotationsbewegung unterhalb der komplementären Teile zu gleiten kommen.

7. Vorrichtung nach Anspruch 6, dadurch gekennzeichnet, dass das axiale Eindringen der mit dem abnehmbaren Kopf (27) (28), (27a) verbundenen Umfangsbundteile in ihre mit dem obersten Teil des mittleren Abschnitts (1) des T verbundene komplementäre Gegenstück (9) (10) aufgrund einer Asymmetrie nur in einer einzigen Winkelposition möglich ist, sodass eine zufällige Umkehrung der Entnahme- und Reinjektionsleitungen vermieden wird und ausserdem die Wirkung der Rotationsantriebsmittel (27) (28), (27a) nur beim axialen Eindringen zwischen die Umfangsbundstücke möglich ist.

8. Vorrichtung nach Anspruch 6, dadurch gekennzeichnet, dass die mit dem Kopf (27) (28), (27a) oder dem zentralen Abschnitt (1) des T verbundenen Umfangsbundstücke (46) (47) jeweils mindestens einen winkelförmigen Anschlag (48), der die Rotation auf den exakt gewählten Wert begrenzt, und einen Verriegelungswulst (49) aufweisen.

9. Vorrichtung nach Anspruch 1, dadurch gekennzeichnet, dass während der gleichzeitigen Rotation des abnehmbaren Kopfes (27) (28), (27a) und des Verschluss-Stücks (15), das den gesamten zu implantierenden Teil der Vorrichtung in derselben Richtung mitzunehmen tendiert, ein mit einer Nut (42) versehenes Rotationsblockierstück (40) des mittleren Abschnitts (1) des T um zwei Abflachungen (12) und (13) des damit verbundenen Stücks (8) eingeführt wird, welches Blockierstück durch ein offenes oder geschlossenes Loch (41), in das der Patient einen Finger, vorzugsweise den Zeigefinger, stecken kann, immobilisiert wird. In der maximalen Versenkposition des Blockierstücks (40) kommt eine zweite Nut (43) desselben in Überdeckung des obersten Teils der mit dem abnehmbaren Kopf (27) (28), (27a) verbundenen Kreiskrone (44), sodass das axiale Zurückspringen des Kopfes während der ablaufenden Vorgänge vor dessen Verriegelung untersagt wird.

10. Vorrichtung nach Anspruch 9, dadurch gekennzeichnet, dass der abnehmbare Kopf aus einem einzigen Stück (27a) besteht, an das zwei Aussenleitungen (35) und (36) geklebt sind, welche ausreichend nachgiebig sind, um die 90°ige Rotation während der Verriegelungsphase mitzumachen, oder aus zwei Stücken (27) (28), von denen das erste (27), das für die Drehung um 90° im mittleren Abschnitt (1) des T während der Verriegelung einer von den Fingern auf die Unebenheiten (34) des obersten Teils ausgeübten Bewegung bestimmt ist, zwei kreisförmige Nuten (30) und (31) besitzt, die jeweils mit einem der beiden Entnahme (32) und Reinjektions- (33) Leitungen verbunden sind, und von denen das zweite (28) mit zwei Aussenleitungen (35) und (36) verbunden ist und die Rotation des Drehteils (27) in seinem Mittelpunkt gestatten, wobei jedoch eine ausreichende Dichtheit zwischen der Leitung (35) und der Nut (30) und zwischen der Leitung (36) und der Nut (31) gegeben ist.

11. Vorrichtung nach einem der vorhergehenden Ansprüche, dadurch gekennzeichnet, dass ein Stöpsel (14) aus vorzugsweise biokompatiblem, elastomerem Material, wie z.B. Polyurethan, eine derartige Form hat, dass er, einmal in Lage gebracht, keinen freien Raum in den Hohlräumen des Verschluss-Stücks (15) lässt, sodass ein Verbleiben aller Fluide vermieden wird.

Fig. 1

Fig. 2

Fig. 4

Fig. 3

Fig. 5

Fig. 6

Fig. 7

Fig. 8

Fig. 9

Fig. 10

Fig. 11

Fig. 12

Fig. 13

Fig. 15

Fig. 14

Fig. 16

Fig. 17

Fig. 20

Fig. 18

Fig. 19

Fig. 21

Fig. 22

Fig. 24     Fig. 23     Fig. 25

Fig. 26